# EUROPEAN PATENT SPECIFICATION

(11) **EP 4 186 417 B1**
(45) Date of publication and mention of the grant of the patent: **12.11.2025**
(21) Application number: 21210258.6
(22) Date of filing: 24.11.2021
(51) Int. Cl.: A61B 5/026, A61B 5/055

(54) **CORONARY ARTERY STENOSIS QUANTIFICATION USING 3D FLOW MRI**
QUANTIFIZIERUNG VON CORONARARTERIENSTENOSE MITTELS 3D-FLUSS-MRT
QUANTIFICATION DE LA STÉNOSE DES ARTÈRES CORONAIRES À L'AIDE D'UN IRM DE FLUX 3D

(43) Date of publication of application: 31.05.2023
(73) Proprietor: Siemens Healthineers AG, 91301 Forchheim (DE)
(72) Inventor: Giese, Daniel, 91052 Erlangen (DE)
(74) Representative: Siemens Healthineers Patent Attorneys

(56) References cited:
- US-B1- 6 408 201
- MICHAEL MARKL ET AL: "4D flow MRI", JOURNAL OF MAGNETIC RESONANCE IMAGING, vol. 36, no. 5, 22 October 2012 (2012-10-22), US, pages 1015 - 1036, XP055222904, ISSN: 1053-1807, DOI: 10.1002/jmri.23632
- KADBI MO ET AL: "Assessment of flow and hemodynamics in the carotid artery using a reduced TE 4D flow spiral phase-contrast MRI", ENGINEERING IN MEDICINE AND BIOLOGY SOCIETY (EMBC), 2013 34TH ANNUAL INTERNATIONAL CONFERENCE OF THE IEEE, IEEE, 3 July 2013 (2013-07-03), pages 1100 - 1103, XP032489092, ISSN: 1557-170X, [retrieved on 20130925], DOI: 10.1109/EMBC.2013.6609697
- ALEX FRYDRYCHOWICZ ET AL: "Four-dimensional phase contrast magnetic resonance angiography: Potential clinical applications", EUROPEAN JOURNAL OF RADIOLOGY, ELSEVIER SCIENCE, NL, vol. 80, no. 1, 29 December 2010 (2010-12-29), pages 24 - 35, XP028287630, ISSN: 0720-048X, [retrieved on 20110128], DOI: 10.1016/J.EJRAD.2011.01.094
- MARKL MICHAEL ET AL: "Comprehensive 4D velocity mapping of the heart and great vessels by cardiovascular magnetic resonance", JOURNAL OF CARDIOVASCULAR MAGNETIC RESONANCE, BIOMED CENTRAL LTD, LONDON UK, vol. 13, no. 1, 14 January 2011 (2011-01-14), pages 7, XP021088949, ISSN: 1532-429X, DOI: 10.1186/1532-429X-13-7

## Description

### Technical field

The present application relates to a method for determining a flow parameter in a coronary artery of the heart. Furthermore, the magnetic resonance imaging system is provided configured to determine the flow parameter, a computer program comprising program code and a carrier comprising the computer program.

### Background

Coronary Artery Disease (CAD) is caused by plaque in the coronary arteries that lead to a stenosis (narrowing) and may lead to a block of blood flow which could lead to e.g. a Heart Attack. CAD is the most common cardiovascular disease and is amongst the main cause of death worldwide. Its economic burden is huge. A non-invasive diagnosis and quantification of a stenosis remains unreliable, especially because the decision to invasively treat a stenosis is directly related to a quantification of the hemodynamic relevance (blood flow and pressure) which can only be insufficiently measured non-invasively.

Currently, the hemodynamic relevance of a stenosis can be assessed in the following way.
- Invasively, under X-RAY (Angiography), pressure measurements can be taken distal and proximal of the stenosis by inserting a catheter into the artery. These pressure measurements define the so-called Fractional Flow Reserve (FFR). A cut-off value (in the order of 0.8) is defined, over which the stenosis are deemed 'hemodynamically relevant', leading to a decision to treat the stenosis (using a stent, for instance)
- Non-invasively using cardiovascular magnetic resonance imaging (CMR), a cardiac perfusion acquisition can be performed (under stress). A perfusion defect of the myocardium is an indirect measurement of a hemodynamic relevant stenosis. In this case, the coronary artery is not measured directly, but only the effect of a potential blood flow block is measured in the heart muscle. This measurement is typically only performed in 2D due to scan time limitations.
- Virtual FFR: Based on CT angiography and computational fluid dynamic simulations, a virtual FFR can be calculated. The CT needs ionizing radiation and the calculation of the Virtual FFR is based on many assumptions and boundary conditions which are not measured. It therefore includes many unknowns (such as pressure and flow) for the derivation of a hemodynamic parameter which increases the inaccuracy of this measure.

The following publications provide solutions for phase contrast MRI in order to quantify blood flow in the human vascular system in a time-resolved manner:
Markl Michael et al: "4D flow MRI", JOURNAL OF MAGNETIC RESONANCE IMAGING, vol. 36, no. 5, October 2012, pages 1015-1036, ISSN: 1053-1807, DOI: 10.1002/jmri. 23632
Kadbi Mo et al: "Assessment of flow and hemodynamics in the carotid artery using a reduced TE 4D flow spiral phase-contrast MRI", ENGINEERING IN MEDICINE AND BIOLOGY SOCIETY (EMBC), 2013 34TH ANNUAL INTERNATIONAL CONFERENCE OF THE IEEE, July 2013, pages 1100-1103, ISSN: 1557-170X, DOI: 10.1109/EMBC.2013.6609697
Alex Frydrychowicz et al: "Four-dimensional phase contrast magnetic resonance angiography: Potential clinical applications", EUROPEAN JOURNAL OF RADIOLOGY, ELSEVIER SCIENCE, NL, vol. 80, no. 1, December 2010, pages 24-35, ISSN: 0720-048X, DOI: 10.1016/J.EJRAD. 2011.01 .094
Markl Michael et al: "Comprehensive 4D velocity mapping of the heart and great vessels by cardiovascular magnetic resonance", JOURNAL OF CARDIOVASCULAR MAGNETIC RESONANCE, BIOMED CENTRAL LTD, LONDON UK, vol. 13, no. 1, January 2011, ISSN: 1532-429X, DOI: 10.1186/1532-429X-13-7

### Summary

Accordingly, a need exists to provide a reliable method to assess the hemodynamic relevance of a stenosis in the coronary arteries of the heart.

This need is met by the features of the independent claims. Further aspects are described in the dependent claims.

According to a first aspect, a method for determining a flow parameter in a coronary artery of the heart is provided. The method comprises, at a magnetic resonance imaging system the steps of determining a heartbeat of the heart. Furthermore, MR signals of the heart are detected taking into account the heartbeat using a 3D imaging sequence of a region of interest comprising the heart, wherein the 3D imaging sequence is a segmented imaging sequence comprising flow encoding segments in which additional flow encoding gradients are switched in different gradient directions, wherein the 3D imaging sequence furthermore comprises additional segments without additional flow encoding gradients. A plurality of 3D k-space data sets are generated from the detected MR signals which were detected in the additional segments and the flow encoding segments. Furthermore at least one morphological MR image of the heart is generated showing at least one coronary artery of the heart based on the detected MR signals. Furthermore a quantitative flow parameter of blood flowing in the coronary artery is determined taking into account the flow encoding segments and the additional segments.

This method allows direct, noninvasive measurement of flow in the coronary artery together with the generation of a morphological image of the coronary artery.

Preferably, the additional flow encoding gradients are switched in three different directions, so that one 3D flow k-space data set is obtained for each of the gradient directions and one additional 3D k-space data set is obtained without the additional flow encoding gradients. The quantitative flow parameter is determined based on the 3D flow k-space data sets and the one additional 3D k-space data set.

Preferably, in each of the flow encoding segments and the additional segments which are acquired in an interleaved way, one k-space line of the corresponding k-space data set is acquired. Preferably this is done for each velocity direction so that one k-space line is acquired in the acquisition block, the flow encoding segment, for each of three velocity directions.

The quantitative flow parameter can be a hemodynamic parameter in the at least one coronary artery.

The quantitative flow parameter can include the determination of a flow vector including a magnitude and the direction of the blood flow in a three-dimensional space, preferably per pixel in the generated image, which can be the morphological image. The quantitative flow parameter can include a Fractional Flow Reserve, FFR.

The at least one morphological MR image showing the coronary artery can be generated based on the 3D flow k-space data sets and the one additional 3D k-space data set. Compared to a normal 3D imaging sequence without the flow encoding segments, this means that there are four times more k-space data points available for the image reconstruction. Accordingly it is possible to either increase the quality such as the signal-to-noise ratio of the morphological image, or it is possible to generate the MR image based on only partially acquired k-space data sets so that the image acquisition time can be reduced. As the flow information and the morphological image are both generated from the same data sets, a registration of the flow parameter to the image data is not necessary.

This can mean that each of the 3D flow k-space data sets and the additional 3D k-space data set is not fully sampled and the at least one morphological MR image is generated from a combined k-space data set which combines the undersampled data sets.

The at least one morphological MR image can be generated using a compressed sensing technology or any other constrained reconstruction scheme. This allows to keep the total acquisition time in an acceptable range such as below 10 minutes.

According to the invention, the acquisition of the MR signals is limited to a diastolic phase of a cardiac cycle of the heart, at least for the flow encoding segments. Preferably, the acquisition of the MR signals is limited to the diastolic phase for all segments of the image acquisition. In the diastolic phase the coronary arteries have the maximum blood flow and furthermore the movement of the heart is comparatively low in this phase of the cardiac cycle.

The quantitative flow parameter is obtained as spatially resolved flow parameter obtained at least for a part of the coronary artery.

Preferably, both the spatially resolved quantitative flow parameter and the at least one morphological MR images are both generated from the 3D flow k-space data sets and the additional 3D k-space data set so that the spatially resolved quantitative flow parameter and at least one morphological MR image are registered to each other. The encoding of the magnitude MR signals and the phase which is used for the flow signal is achieved simultaneously so that a precise location of the flow parameter is possible.

Furthermore, the corresponding magnetic resonance imaging system is provided comprising a control module which is configured to carry out the method mentioned above or as discussed in further detail below.

Additionally a computer program comprising program code to be executed by the at least one control module of the magnetic resonance imaging system is provided wherein the execution of the program code causes the imaging system to carry out a method as mentioned above or as discussed in detail below.

Last but not least, a carrier comprising the computer program is provided wherein the carrier is one of an electronic signal, optical signal, radio signal, and computer readable storage medium.

### Brief description of the drawings

The foregoing and additional features and effects of the invention will become apparent from the following detailed description when read in conjunction with the accompanying drawings in which like reference numerals refer to like elements.
Fig. 1 shows a schematic view of an MR imaging system configured to determine a motion related parameter of the heart.
Fig. 2 shows a schematic view of sequence diagram of an imaging sequence allowing the coronary artery flow to be determined.
Fig. 3 shows a schematic view of a flowchart comprising the steps carried out to determine the flow in the coronary artery.

### Detailed description

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are to be illustrative only.

The drawings are to be regarded as being schematic representations and elements illustrated in the drawings are not necessarily shown to scale. Rather, the various elements are represented such, that their function in general purpose becomes apparent to a person with skill in the art. Any connection or coupling between functional blocks, devices, components of physical or functional units shown in the drawings and described hereinafter may be implemented by an indirect or direct connection. A coupling between components may be established over a wired or wireless connection. Functional blocks may be implemented in hardware, software, firmware, or a combination thereof.

The invention allows the direct, non-invasive measurement of the hemodynamic relevance of a coronary artery stenosis. It combines and adapts several CMR techniques, namely CMR-based coronary angiography and CMR-based flow quantification using phase-contrast MRI.

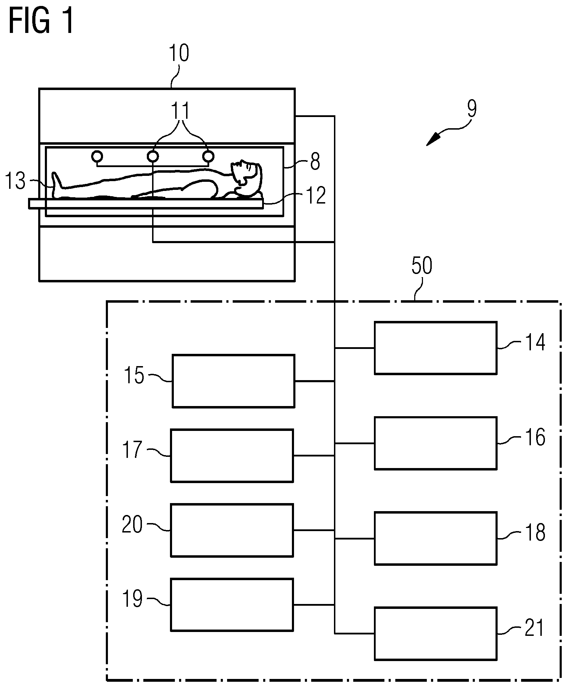

Fig. 1 shows a schematic view of an MR imaging system 9, which comprises a magnet 10 generating the magnetic field B0. The patient or object under examination 13 lying on a table 12 is moved into the center of the MR imaging system 9, where the MR signals can be detected after excitation by RF pulses using coils 11. By applying RF pulses and magnetic field gradients, the nuclear spins of object 13, especially the part located in the receiving coils are excited and location coded currents induced by relaxation can be detected. The way how MR images, especially 3D image data sets are generated and how the MR signals are detected, using a sequence of RF pulses and a sequence of magnetic field gradients, is known in the art, so that a detailed general explanation thereof is omitted. The MR system may furthermore comprise shim coils 8 which are used to correct in-homogeneities of the magnetic field B0.

The MR imaging system 9 comprises a control module 50 which is used for controlling the MR imaging system. The control module 50 comprises a gradient control unit 14 for controlling and switching the magnetic field gradients, an RF control unit 15 for controlling and generating RF pulses for the imaging sequences. The image sequence control unit 16 is provided to control the sequence of the applied RF pulses and magnetic field gradients and thus is also configured to partly control the gradient control unit 14 and the RF control unit 15. In a memory 17, computer programs needed for operating the MR imaging system and the imaging sequences necessary for generating the MR images can be stored together with the generated MR images. The MR images and any further information can be displayed on a display 18 wherein a human machine interface 19 is provided, which can be used by an operator of the MR imaging system to control the MR imaging system. A flow quantification unit 20 is provided configured to quantify a flow parameter in a coronary artery shown in the generated MR images. Unit 20 can be configured to determine a flow vector pixel-wise so that a stenosis can be identified by comparing the flow vectors from the different pixels. A central processing unit 21 can coordinate the operation of the different functional units shown in Fig. 1 and can comprise one or more processors, which can carry out instructions stored on the memory 17. The memory can include program code to be executed by the processing unit 21. As will be explained below, the control module, especially gradient control unit 14, the RF control unit 15, the image sequence control unit 16, and flow quantification unit 20 can be configured to quantify flow in the coronary artery using 3D k-space data sets of the patient 13 as will be explained in detail further below.

Several investigations are constantly improving coronary angiography using CMR. However, these measurement measure only the morphology of coronary arteries. A potential stenosis can therefore only be diagnosed if a narrowing is visible in the artery. On the other hand, the measurement of time-resolved 3D blood flow velocity using 4D flow can currently not be applied to coronary arteries due to the very high spatial resolution needed as well as due to the strong motion throughout the cardiac cycle of the coronary arteries.

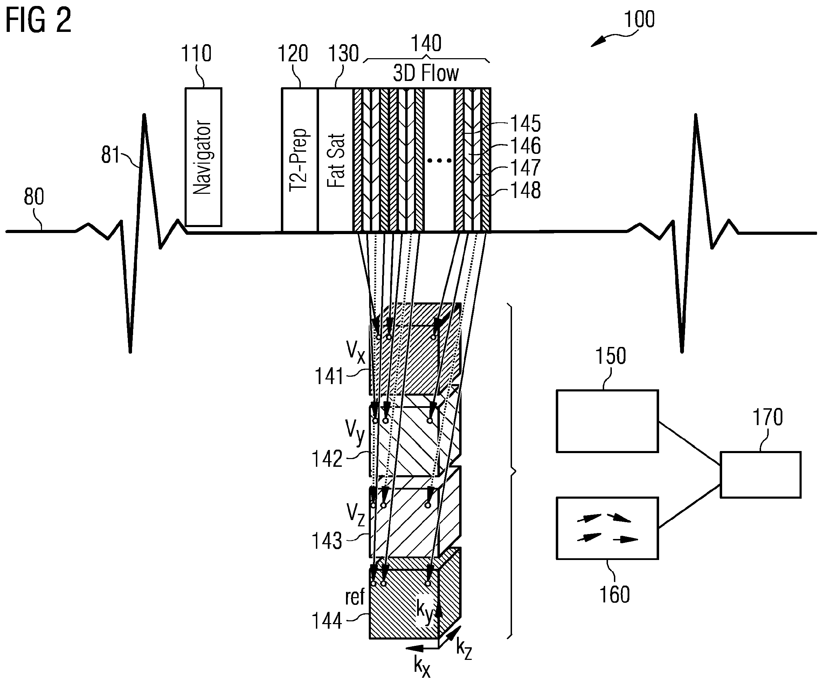

Fig. 2 shows an example of an imaging sequence with which a morphological image of the coronary arteries is possible together with a quantification of the flow in the coronary arteries. The heartbeat of the examined person is monitored such as the ECG signal 80 where the R wave 81 is shown symbolically. The imaging sequence 100 is a 3D flow sequence which can be used to measure the fractional flow reserve, FFR. As the velocity vectors are measured, any related hemodynamic quantity can be deducted from this 3D vector field. These include: maximum flow, flow volume, flow profiles, pressure gradients along the coronary artery, helicity, vorticity, etc. For breathing artifact reduction, a navigator 110 is played out. Furthermore, a T2 contrast preparation module 120 is played out followed by a fat saturation module 130. The 3D flow acquisition block 140 acquires sequentially at least one k-space line in each velocity direction. The flow encoding is obtained with additional encoding gradients which are applied in the three different gradient directions. By way of example in order to determine a flow component in an x-direction, additional gradients are applied in the x-direction. During each of the segments 145-148 at least one k-space line of each velocity direction including of a reference 148 where no additional flow encoding gradient is played out is acquired. This leads to in total four 3D k-space data sets 141-144 wherein each 3D k-space data set comprises the k-space lines of one velocity direction. Accordingly, several cardiac cycles are needed to fill up the different k-space volumes.

A morphological MR image 150 of the heart can be generated using the four different data sets 141-144. The image can be reconstructed using construction techniques such as compressed sensing. As for different image data sets are provided it is possible to further reduce the amount of k-space lines acquired in each data set 141-144. As an alternative, the signal-to-noise ratio could be improved as the morphological image can be reconstructed based on four data sets when these data sets are each fully acquired or the undersampling is at least reduced. Furthermore the data sets 141 to 144 are used to generate an image 160 with spatially resolved blood flow velocity in the coronary arteries or any other artery in the image as symbolized by the arrows in image 160. Images 150 and 160 can be combined to generate an image 170 which is a morphological image which also shows the flow information in the coronary arteries, e.g. the flow information may be color coded into the image.

Preferably the k-space lines are only acquired in a period of the heartbeat in which the coronary arteries have the maximum blood flow, this means that the image segments are acquired during the diastolic period of the cardiac cycle. The described technology can take advantage of all improved coronary angiography CMR techniques such as Fat Sat, prescan motion compensation, resting phase definition etc. This can lead to an optimal contrast in the morphological image of the coronary arteries while additionally measuring the blood flow in 3D through the coronary arteries encoded in the phase of the signal. As all the four data sets are used for the morphological image in the flow determination, both morphology and flow will be automatically registered to each other. The 3D flow CMR measurement therefore has all the advantages of the coronary angiography CMR in addition to directly measuring the hemodynamics in the arteries.

The scan time, as compared to standard coronary angiography is increased by a factor of 4, however, the signal-to-noise ratio is doubled and the similarity of each flow encoding acquisition can lead to a higher acceleration factor which can be used in compressed sensing reconstruction. The proposed technique can measure the blood flow directly and does not need any computational fluid dynamic calculations nor prior assumptions regarding boundary conditions.

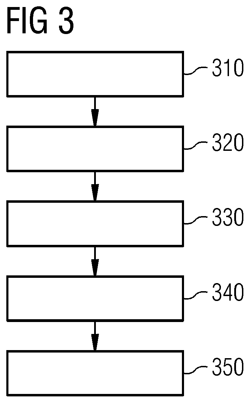

Fig. 3 summarizes some of the steps carried out during the quantitative determination of the flow parameter in the coronary artery. In step 310 the heartbeat of the heart is determined, by way of example using the ECG signal of the heart as shown in Fig. 2. In a further step the MR signals of the heart are detected in step 320 wherein the 3D imaging sequence as discussed in connection with Fig. 2 is used. The imaging sequence is an imaging sequence comprising different segments such as the flow encoding segments 145, 146, 147 in which additional flow encoding gradients are switched in different directions. Furthermore, the imaging sequence comprises a segment 148 without additional flow encoding gradients which are just switched in addition to the other gradients in order to encode flow. In step 330, after several heartbeats the 3D the k-space data sets 141-144 are generated/obtained. In step 340 at least one morphological MR image of the heart is generated which shows one or several coronary arteries of the heart. At the same time it is possible in step 350 to determine the quantitative flow parameter of the blood flow in the coronary artery such as the fractional flow reserve, FFR, however it should be noted that any other flow parameter such as the absolute flow velocity or relative changes may be determined.

Summarizing, an effective and noninvasive way is obtained to obtain at the same time a morphological image of the coronary artery and a quantification of the blood flow in the coronary artery without invasively measuring the blood flow.

## Claims

1. A computer-implemented method for determining a quantitative flow parameter in a coronary artery of a heart, the method comprising at a magnetic resonance imaging system:
- determining a heartbeat of the heart,
- detecting MR signals from the heart, taking into account the heartbeat, using a 3D imaging sequence of a region of interest comprising the heart, wherein the 3D imaging sequence is a segmented imaging sequence comprising flow encoding segments (145-147) in which additional flow encoding gradients are switched in different gradient directions, and additional segments (148) without additional flow encoding gradients,
- generating a plurality of 3D k-space data sets (141-144) from the detected MR signals detected in the additional segments and the flow encoding segments,
- generating at least one morphological MR image (150) of the heart showing at least one coronary artery of the heart based on the detected MR signals,
- determining the quantitative flow parameter of blood flowing in the coronary artery taking into account the flow encoding segments and the additional segments,
**characterized in that** at least for the flow encoding segments, the acquisition of the MR signals is limited to a diastolic phase of a cardiac cycle of the heart.

2. The method of claim 1, wherein the additional flow encoding gradients are switched in three different directions, resulting in one 3D flow k-space data set (141-143) for each of the gradient directions, and one additional 3D k-space data set (144) obtained without the additional flow encoding gradients, wherein the quantitative flow parameter is determined based on the 3D flow k-space data sets and the one additional 3D k-space data set.

3. The method of claim 1 or 2, wherein in each of the flow encoding segments (145-147) and additional segments (148) acquired in an interleaved way, one k-space line of a corresponding 3D k-space data set is acquired.

4. The method of any preceding claim, wherein determining a quantitative flow parameter comprises determining a hemodynamic parameter in the at least one coronary artery.

5. The method of any preceding claim, wherein determining a quantitative flow parameter comprises determining a magnitude and a direction of the blood flow in a three dimensional space.

6. The method of any preceding claim, wherein determining a quantitative flow parameter comprises determining a fractional flow reserve, FFR.

7. The method of any of claims 2 to 6, wherein the at least one morphological MR image (150) is generated based on the three 3D flow k-space data sets (141-143) and the one additional 3D k-space data set (144).

8. The method of claim 7, wherein each of the 3D flow k-space data sets (141-143) and the additional 3D k-space data set (144), is not fully sampled, wherein the at least one morphological MR image is generated from a combined k- space data set combining the 3D flow k-space data sets and the one additional 3D k-space data set.

9. The method of any preceding claim, wherein the at least one morphological MR image is generated using a compressed sensing technology.

10. The method of any preceding claim, wherein the quantitative flow parameter is obtained as spatially resolved flow parameter obtained at least for a part of the coronary arteries.

11. The method of claim 2 and 10, wherein both the spatially resolved quantitative flow parameter and the at least one morphological MR image are both obtained from the three 3D flow k-space data sets and the additional 3D k-space data set, so that the spatially resolved quantitative flow parameter and the at least one morphological MR image are registered to each other.

12. A magnetic resonance imaging system comprising a control module (50) configured to
- determine a heartbeat of the heart,
- detect MR signals from the heart, taking into account the heartbeat, using a 3D imaging sequence to a region of interest comprising the heart, wherein the 3 D imaging sequence is a segmented imaging sequence comprising flow encoding segments (145-147) in which additional flow encoding gradients are switched in different gradient directions, and additional segments (148) without additional flow encoding gradients,
- generate a plurality of 3 D k-space data sets (141-143) from the detected MR signals detected in the additional segments and the flow encoding segments,
- generate at least one morphological MR image (150) of the heart showing at least one coronary artery of the heart based on the detected MR signals,
- determine a quantitative flow parameter of blood flowing in the coronary artery taking into account the flow encoding segments and the additional segments,
**characterized in that** at least for the flow encoding segments, the acquisition of the MR signals is limited to a diastolic phase of a cardiac cycle of the heart.

13. The magnetic resonance imaging system of claim 12, further being configured to carry out a method as mentioned in any of claims 2 to 11.

14. A computer program comprising program code to be executed by the control module of a magnetic resonance imaging system according to claim 12, wherein execution of the program code causes the magnetic resonance imaging system to carry out a method as mentioned in any of claims 1 to 11.

15. A carrier comprising the computer program of claim 14, wherein the carrier is one of an electronic signal, optical signal, radio signal and computer readable storage medium.

## Patentansprüche

1. Computerimplementiertes Verfahren zum Bestimmen eines quantitativen Flussparameters in einer Koronararterie eines Herzens, wobei das Verfahren an einem Magnetresonanz-Bildgebungssystem Folgendes umfasst:
- Bestimmen eines Herzschlags des Herzens,
- Detektieren von MR-Signalen von dem Herzen unter Berücksichtigung des Herzschlags unter Verwendung einer 3D-Bildgebungssequenz einer Interessensregion, die das Herz umfasst, wobei die 3D-Bildgebungssequenz eine segmentierte Bildgebungssequenz ist, die Flusscodierungssegmente (145-147), in denen zusätzliche Flusscodierungsgradienten in unterschiedliche Gradientenrichtungen geschaltet werden, und zusätzliche Segmente (148) ohne zusätzliche Flusscodierungsgradienten umfasst,
- Generieren einer Vielzahl von 3D-k-Raum-Datensätzen (141-144) aus den detektierten MR-Signalen, die in den zusätzlichen Segmenten und den Flusscodierungssegmenten detektiert wurden,
- Generieren mindestens eines morphologischen MR-Bildes (150) des Herzens, das mindestens eine Koronararterie des Herzens zeigt, basierend auf den detektierten MR-Signalen,
- Bestimmen des quantitativen Flussparameters des in der Koronararterie fließenden Blutes unter Berücksichtigung der Flusscodierungssegmente und der zusätzlichen Segmente,
**dadurch gekennzeichnet, dass** mindestens für die Flusscodierungssegmente die Erfassung der MR-Signale auf eine diastolische Phase eines Herzzyklus des Herzens beschränkt ist.

2. Verfahren nach Anspruch 1, wobei die zusätzlichen Flusscodierungsgradienten in drei verschiedene Richtungen geschaltet werden, was zu einem 3D-Fluss-k-Raum-Datensatz (141-143) für jede der Gradientenrichtungen und einem zusätzlichen 3D-k-Raum-Datensatz (144) führt, der ohne die zusätzlichen Flusscodierungsgradienten erhalten wird, wobei der quantitative Flussparameter basierend auf den 3D-Fluss-k-Raum-Datensätzen und dem einen zusätzlichen 3D-k-Raum-Datensatz bestimmt wird.

3. Verfahren nach Anspruch 1 oder 2, wobei in jedem der Flusscodierungssegmente (145-147) und zusätzlichen Segmente (148), die auf eine verschachtelte Weise erfasst werden, eine k-Raum-Linie eines entsprechenden 3D-k-Raum-Datensatzes erfasst wird.

4. Verfahren nach einem der vorhergehenden Ansprüche, wobei Bestimmen eines quantitativen Flussparameters Bestimmen eines hämodynamischen Parameters in der mindestens einen Koronararterie umfasst.

5. Verfahren nach einem der vorhergehenden Ansprüche, wobei Bestimmen eines quantitativen Flussparameters Bestimmen einer Größe und einer Richtung des Blutflusses in einem dreidimensionalen Raum umfasst.

6. Verfahren nach einem der vorhergehenden Ansprüche, wobei Bestimmen eines quantitativen Flussparameters Bestimmen einer fraktionellen Flussreserve, FFR, umfasst.

7. Verfahren nach einem der Ansprüche 2 bis 6, wobei das mindestens eine morphologische MR-Bild (150) basierend auf den drei 3D-Fluss-k-Raum-Datensätzen (141-143) und dem einen zusätzlichen 3D-k-Raum-Datensatz (144) generiert wird.

8. Verfahren nach Anspruch 7, wobei jeder der 3D-Fluss-k-Raum-Datensätze (141-143) und der zusätzliche 3D-k-Raum-Datensatz (144) nicht vollständig abgetastet wird, wobei das mindestens eine morphologische MR-Bild aus einem kombinierten k-Raum-Datensatz generiert wird, der die 3D-Fluss-k-Raum-Datensätze und den einen zusätzlichen 3D-k-Raum-Datensatz kombiniert.

9. Verfahren nach einem der vorhergehenden Ansprüche, wobei das mindestens eine morphologische MR-Bild unter Verwendung einer komprimierten Abfühltechnologie generiert wird.

10. Verfahren nach einem der vorhergehenden Ansprüche, wobei der quantitative Flussparameter als räumlich aufgelöster Flussparameter erhalten wird, der mindestens für einen Teil der Koronararterien erhalten wird.

11. Verfahren nach Anspruch 2 und 10, wobei sowohl der räumlich aufgelöste quantitative Flussparameter als auch das mindestens eine morphologische MR-Bild beide aus den drei 3D-Fluss-k-Raum-Datensätzen und dem zusätzlichen 3D-k-Raum-Datensatz erhalten werden, so dass der räumlich aufgelöste quantitative Flussparameter und das mindestens eine morphologische MR-Bild miteinander registriert werden.

12. Magnetresonanz-Bildgebungssystem, umfassend ein Steuermodul (50), das ausgelegt ist zum:
- Bestimmen eines Herzschlags des Herzens,
- Detektieren von MR-Signalen von dem Herzen unter Berücksichtigung des Herzschlags unter Verwendung einer 3D-Bildgebungssequenz einer Interessensregion, die das Herz umfasst, wobei die 3D-Bildgebungssequenz eine segmentierte Bildgebungssequenz ist, die Flusscodierungssegmente (145-147), in denen zusätzliche Flusscodierungsgradienten in unterschiedliche Gradientenrichtungen geschaltet werden, und zusätzliche Segmente (148) ohne zusätzliche Flusscodierungsgradienten umfasst,
- Generieren einer Vielzahl von 3D-k-Raum-Datensätzen (141-143) aus den detektierten MR-Signalen, die in den zusätzlichen Segmenten und den Flusscodierungssegmenten detektiert wurden,
- Generieren mindestens eines morphologischen MR-Bildes (150) des Herzens, das mindestens eine Koronararterie des Herzens zeigt, basierend auf den detektierten MR-Signalen,
- Bestimmen eines quantitativen Flussparameters des in der Koronararterie fließenden Blutes unter Berücksichtigung der Flusscodierungssegmente und der zusätzlichen Segmente;
**dadurch gekennzeichnet, dass** mindestens für die Flusscodierungssegmente die Erfassung der MR-Signale auf eine diastolische Phase eines Herzzyklus des Herzens beschränkt ist.

13. Magnetresonanz-Bildgebungssystem nach Anspruch 12, das ferner dazu ausgelegt ist, ein Verfahren nach einem der Ansprüche 2 bis 11 auszuführen.

14. Computerprogramm, umfassend Programmcode, der durch das Steuermodul eines Magnetresonanz-Bildgebungssystems nach Anspruch 12 auszuführen ist, wobei die Ausführung des Programmcodes bewirkt, dass das Magnetresonanz-Bildgebungssystem ein Verfahren nach einem der Ansprüche 1 bis 11 ausführt.

15. Träger, umfassend das Computerprogramm nach Anspruch 14, wobei der Träger eines von einem elektronischen Signal, einem optischen Signal, einem Funksignal und einem computerlesbaren Speichermedium ist.

## Revendications

1. Un procédé mis en œuvre par ordinateur de détermination d'un paramètre quantitatif d'écoulement dans l'artère coronaire d'un cœur, le procédé comprenant, à un système d'imagerie par résonnance magnétique :
- déterminer un battement cardiaque du cœur,
- détecter des signaux RM du cœur, en prenant en compte le battement cardiaque, en utilisant une séquence d'imagerie en 3D d'une région à laquelle on s'intéresse comprenant le cœur, dans lequel la séquence d'imagerie en 3D est une séquence d'imagerie segmentée comprenant des segments (145-147) codant l'écoulement, dans lesquels des gradients supplémentaires de codage de l'écoulement sont commutés dans différentes directions de gradient, et des segments (148) supplémentaires sans gradient supplémentaire de codage de l'écoulement,
- créer une pluralité d'ensembles (141-144) de données de l'espace k en 3D à partir des signaux RM détectés détectés dans les segments supplémentaires et les segments de codage de l'écoulement,
- créer au moins une image (150) RM morphologique du cœur montrant au moins une artère coronaire du cœur sur la base des signaux RM détectés,
- déterminer le paramètre quantitatif d'écoulement de l'écoulement du sang dans l'artère coronaire en prenant en compte les segments de codage de l'écoulement et les segments supplémentaires,
**caractérisé en ce qu'**au moins pour les segments codant l'écoulement, l'acquisition des signaux RM est limitée à une phase diastolique d'un cycle cardiaque du cœur.

2. Le procédé de la revendication 1, dans lequel les gradients supplémentaires codant l'écoulement sont commutés dans trois directions différentes, ce qui se traduit par un ensemble (141-143) de données dans l'espace k d'écoulement en 3D pour chacune des directions de gradient, et un ensemble (144) supplémentaire de données dans l'espace k en 3D obtenu sans les gradients supplémentaires codant l'écoulement, dans lequel le paramètre quantitatif d'écoulement est déterminé sur la base des ensembles de données dans l'espace k d'écoulement en 3D et du un ensemble supplémentaire de données dans l'espace k en 3D.

3. Le procédé de la revendication 1 ou 2, dans lequel, dans chacun des segments (145-147) de codage de l'écoulement et des segments (148) supplémentaires acquis d'une façon imbriquée, une ligne dans l'espace k d'un ensemble de données dans l'espace k en 3D correspondant est acquise.

4. Le procédé de l'une quelconque des revendications précédentes, dans lequel déterminer un paramètre quantitatif d'écoulement comprend déterminer un paramètre hémodynamique dans la au moins une artère coronaire.

5. Le procédé de l'une quelconque des revendications précédentes, dans lequel déterminer un paramètre quantitatif d'écoulement comprend déterminer une amplitude et une direction du courant sanguin dans un espace en trois dimensions.

6. Le procédé de l'une quelconque des revendications précédentes, dans lequel déterminer un paramètre quantitatif d'écoulement comprend déterminer une réserve fractionnée d'écoulement, FRR.

7. Le procédé de l'une quelconque des revendications 2 à 6, dans lequel la au moins une image (150) RM morphologique est créée sur la base des trois ensembles (141-143) et du un ensemble (144) supplémentaire de données dans l'espace k en 3D.

8. Le procédé de la revendication 7, dans lequel chacun des ensembles (141-143) de données dans l'espace k d'écoulement en 3D et l'ensemble (144) supplémentaire de données dans l'espace k en 3D, n'est pas échantillonné complètement, dans lequel la au moins une image RM morphologique est créée à partir d'un ensemble combiné de données dans l'espace k combinant les ensembles de données dans l'espace k d'écoulement en 3D et le un ensemble supplémentaire de données dans l'espace k en 3D.

9. Le procédé de l'une quelconque des revendications précédentes, dans lequel la au moins une image RM morphologique est créée en utilisant une technologie de détection comprimée.

10. Le procédé de l'une quelconque des revendications précédentes, dans lequel le paramètre quantitatif d'écoulement est obtenu sous la forme d'un paramètre d'écoulement résolu spatialement obtenu au moins pour une partie des artères coronaires.

11. Le procédé de la revendication 2 et 10, dans lequel, à la fois le paramètre quantitatif d'écoulement résolu spatialement et la au moins une image RM morphologique sont obtenus à partir des trois ensembles de données dans l'espace k d'écoulement en 3D et de l'ensemble supplémentaire de données dans l'espace k en 3D, de sorte que le paramètre quantitatif d'écoulement résolu spatialement et la au moins une image RM morphologique se correspondent l'un à l'autre.

12. Un système d'imagerie par résonnance magnétique comprenant un module (50) de commande configuré pour
- déterminer un battement cardiaque du cœur,
- détecter des signaux RM du cœur, en prenant en compte le battement cardiaque, en utilisant une séquence d'imagerie en 3D d'une région à laquelle on s'intéresse comprenant le cœur, dans lequel la séquence d'imagerie en 3D est une séquence d'imagerie segmentée comprenant des segments (145-147) codant l'écoulement, dans lesquels des gradients supplémentaires de codage de l'écoulement sont commutés dans différentes directions de gradient, et des segments (148) supplémentaires sans gradient supplémentaire de codage de l'écoulement,
- créer une pluralité d'ensembles (141-143) de données de l'espace k en 3D à partir des signaux RM détectés détectés dans les segments supplémentaires et les segments de codage de l'écoulement,
- créer au moins une image (150) RM morphologique du cœur montrant au moins une artère coronaire du cœur sur la base des signaux RM détectés,
- déterminer un paramètre quantitatif d'écoulement du sang s'écoulant dans l'artère coronaire en prenant en compte les segments codant l'écoulement et les segments supplémentaires,
**caractérisé en ce qu'**au moins pour les segments codant l'écoulement, l'acquisition des signaux RM est limitée à une phase diastolique d'un cycle cardiaque du cœur.

13. Le système d'imagerie par résonnance magnétique de la revendication 12, configuré en outre pour effectuer un procédé tel que mentionné à l'une quelconque des revendications 2 à 11.

14. Un programme d'ordinateur comprenant un code de programme à exécuter par le module de commande d'un système d'imagerie par résonnance magnétique suivant la revendication 12,
dans lequel l'exécution du code de programme fait que le système d'imagerie par résonnance magnétique exécute un procédé tel que mentionné à l'une quelconque des revendications 1 à 11.

15. Un support comprenant le programme d'ordinateur de la revendication 14, dans lequel le support de l'un d'un signal électronique, d'un signal optique, d'un signal radio et d'un support de mémoire déchiffrable par ordinateur.
